# EUROPEAN PATENT SPECIFICATION

(11) **EP 1 000 027 B1**
(45) Date of publication and mention of the grant of the patent: **02.04.2003**
(21) Application number: 98937576.1
(22) Date of filing: 18.07.1998
(51) Int. Cl.: C07D 209/88, A61K 31/40

(54) **THERMODYNAMICALLY STABLE MODIFICATION OF 1-(4-CARBAZOLYLOXY)-3- 2-(2-METHOXYPHENOXY)ETHYLAMINO]-2-PROPANOLE, PROCESS FOR ITS PREPARATION AND PHARMACEUTICAL COMPOSITIONS CONTAINING IT**
THERMODYNAMISCH STABILE MODIFIZIERUNG VON 1-(4-CARBAZOLYLOXY)-3- [2-(2-METHOXYPHENOXY)ETHYLAMINO]-2-PROPANOL, VERFAHREN ZU IHRER HERSTELLUNG UND IHRE ENTHALTENDE PHARMAZEUTISCHE ZUSAMMENSETZUNGEN
MODIFICATION THERMODYNAMIQUEMENT STABLE DE 1-(4-CARBAZOLYLOXY)-3- 2-(2-METHOXYPHENOXY)ETHYLAMINO]-2-PROPANOLE, SON PROCEDE DE PREPARATION ET COMPOSITIONS PHARMACEUTIQUES LE CONTENANT

(30) Priority: 22.07.1997 EP 97112491
(43) Date of publication of application: 17.05.2000
(73) Proprietor: Roche Diagnostics GmbH, 68298 Mannheim (DE)
(72) Inventor: REINHOLZ, Erhard, D-68542 Heddesheim (DE); BEYER, Peter, D-67433 Neustadt (DE)
(74) Representative: Witte, Hubert, Dr.
(86) International application number: EP9804475
(87) International publication number: WO99005105

(56) References cited:
- EP-A- 0 127 099
- DE-A- 2 815 926
- G. Z. FEUERSTEIN ET. AL.: "carvedilol update III. Rationale for use in congestive heart failure." DRUGS TODAY, vol. 31F, September 1995, pages 1-23, XP002039368

## Description

The present invention relates to a new thermodynamically stable modification of (±)1-(4-carbazolyloxy)-3-[2-(2-methoxyphenoxy)ethylamino]-2-propanole (Carvedilol), pharmacologically acceptable salts or optically active forms thereof, processes for the preparation, and pharmaceutical compositions containing it.

Carvedilol, having a melting point of 114-115°C. is a compound with excellent pharmacological properties (Merck Index 1 1. Ed. No. 1882), known to be active in the treatment of cardiac diseases. The preparation and its use in medicine is described in EP-B-0 004 920.

Carvedilol has a chiral center and, as such, can exist either as individual stereoisomers or in racemic form. Both the racemate and stereoisomers may be obtained according to procedures well known in the art (EP-B-0127099).

It has now been discovered that Carvedilol can be isolated in two different modifications depending upon the method of preparation which are distinguishable by their infra-red Raman and X-ray powder diffraction spectra, and their melting points. The two polymorphic forms are monotropic and they are hereinafter designated as Form I and Form II. It is desirable to prepare a therapeutic agent consisting of an unique and defined composition which has a high storage stability.

The present invention provides a thermodynamically stable crystalline form of Carvedilol substantially free of other physical forms having a melting point about 123-126°C, and an infra-red spectrum with a sharp peak at 3451 cm⁻¹, which is referred to hereinafter as Form I.

The invention also provides a process for producing this substantially pure Form I. In another embodiment of this invention, there is provided a pharmaceutical formulation containing the substantially pure Form I of Carvedilol as an active ingredient.

Finally, the present invention provides a method of using the new substantially pure form to prevent and/or treat circulatory and cardiac diseases.

Where reference is made in this application to Form I or Form II substantially free of other physical forms, it preferably means that at least 90% by weight of Form I or Form II is present in that modification.

Form II is the modification of Carvedilol prepared and purified according to EP-B-0 004 920.

Surprisingly it was now found that a new thermodynamically stable modification of Carvedilol (Form 1) with a higher melting point is obtained when the process of manufacture is slightly altered.

The melting points of each Forms I respectively II depend upon their level of purity, consequently Form I has been found to have a melting point of about 123-126°C, Form II about 114-115°C.

Furtheron it has been discovered that Form 1 is that of being the thermodynamically stable form, which is of advantage. Therefore this thermodynamically stable form is given preference in the preparation of pharmaceutical formulations.

Pharmaceutically acceptable salts are considered to be encompassed within the compounds and the method of the present invention. The term "pharmaceutically acceptable salts" refers to salts of substantially pure Form I which are substantially non-toxic to living organism. For the conversion of Carvedilol into its pharmacologically acceptable salts, it is reacted, preferably in an organic solvent, with an equivalent amount of an inorganic or organic acid, for example hydrochloric acid, hydrobromic acid, phosphoric acid. sulphuric acid. acetic acid. citric acid, maleic acid or benzoic acid. It should be recognized that any particular anion forming a part of any salt of this invention is not critical, so long as the salt, as a whole, is pharmacologically acceptable and as long as the anion moiety does not contribute undesired qualities.

For the resolution of the racemates, there can be used for example, tartaric acid, malic acid, camphoric acid or camphorsulphonic acid.

According to another aspect, the invention provides a pharmaceutical composition. which comprises Form I substantially free of other physical forms and a pharmaceutical acceptable carrier or adjuvant.

The compounds of the present invention may be administered by any suitable route, preferably in the form of a pharmaceutical composition adapted to such a route and in dose effective for the treatment intended. Therapeutically effective doses of the compounds of the present invention required to prevent or arrest the progress of the medical condition are readily ascertained by one of ordinary skill in the art.

Accordingly, the invention provides a class of novel pharmaceutical compositions comprising Carvedilol of Form I of the present invention, in association with one or more non-toxic pharmaceutically acceptable carriers and/or adjuvants (collectively referred to herein as "carrier materials") and, if desired, other active ingredients. The compounds and compositions may, for example, be administered orally. intravascularly, intraperitoneally, subcutaneously, intramuscularly or topically.

For all administrations, the pharmaceutical composition may be in the form of, for example, a tablet, capsule, creme, ointment, gel, lotion, suspension or liquid. The pharmaceutical composition is preferably made in the form of a dosage unit containing a particular amount of the active ingredient. Examples of such dosage units are tablets or capsules. A suitable daily dose for a mammal may vary widely depending on the condition of the patient and other factors. However, a dose from about 0.01 to 100 mg/kg body weight, particularly from about 0.05 to 3 mg/kg body weight, respectively 0.01-10 mg/cm² skin, may be appropriate. The active ingredient may also be administered by injection.

The dose regimen for treating a disease condition with the compounds and/or compositions of this invention is selected in accordance with a variety of factors, including the type, age, weight, sex and medical conditions of the patient and in accordance to the severity of the desease and thus may vary widely.

For therapeutic purposes, the compounds of the invention are ordinarily combined with one ore more adjuvants appropriate to the indicated route of administration. If per os, the compound may be admixed with lactose, sucrose, starch powder, cellulose esters of alkanoic acids, cellulose alkyl ester, talc, stearic acid, magnesium stearat, magnesium oxide, sodium and calcium salts of phosphoric and sulphuric acids, gelatine, acacia, sodium alginate, polyvinyl-pyrrolidone and/or polyvinyl alcohol, and thus tabletted or encapsulated for convenient administration. Alternatively, the compound may be dissolved in water, polyethylene glycol, propylene glycol, ethanol, corn oil, cotton seed oil, peanut oil, sesarn oil, benzyl alcohol, sodium chloride and/or various buffers. Appropriate additives for the use as ointments. cremes or gels are for example paraffine, vaseline, natural waxes, starch, cellulose, or polyethylenglycole. Other adjuvants and modes of administration are well and widely known in the pharmaceutical art.

Appropriate dosages in any given instance, of course, depend upon the nature and severity of the condition treated, the route of administration and the species of mammal involved, including its size and any individual idiosyncracies.

Representative carriers, dilutions and adjuvants include, for example, water, lactose, gelatine starch, magnesium stearate, talc, vegetable oils, gums, polyalkylene glycols, petroleum gelly, etc. The pharmaceutical compositions may be made up in a solid form, such as granules, powders or suppositories, or in liquid form, such as solutions, suspensions or emulsions. The pharmaceutical compositions may be subjected to conventional pharmaceutical adjuvants, such as preservatives, stabilizers, wetting agents, emulsifiers, buffers, etc.

As indicated, the dose administered and the treatment regimen will be dependent, for example, on the disease, the severity thereof, on the patient being treated and his response to treatment and, therefore, may be widely varied.

### Characterization of Forms I and II of Carvedilol

### Thermomicroscopy

Thermal analysis was carried out with a Kofler heating stage (Reichert, Vienna) mounted on a video-equipped Olympus microscope BH-2 or with a Kofler heating stage microscope Thermovat® (Reichert, Vienna); both microscopes with polarisation facility and digital thermometer.

Form II consists of heterogeneously looking rhombohedral to hexagonally shaped lamellar crystals up to a size of 120 µm, which melt about 114-115°C, whereas Form 1 consists of 40 µm large grains, respectively prisms, which melt about 123-126 °C.

### Differential Scanning Calorimetry (DSC)

DSC was carried out with DSC-7 (Perkin-Elmer, Norwalk, Ct., USA) equipped with cooling system CCA-7, perforated Al sample capsules (25 µl), weighed object 1.5 mg each (ultra-micro weighing scale UM 3, Mettler, CH-Greifensee, Switzerland). Nitrogen 4.0 as flushing gas (20 ml min⁻¹). Computer-aided recording of DSC signal. Calibration of temperature indication for CCA curves with melting point for water and caffeine anhydrate (melting point 236.2 °C), each with tightly sealed sample capsule. Calibration of ordinates (DSC signal) with melting heat of indium 99.999% (Perkin-Elmer, Norwalk, Ct., USA).

The measured melting points correspond to the ones determined thermomicroscopically. It could be estimated by the way of the measured melting heats (Form 1: ΔH_{f} 48.2 kJ/mol, Form II ΔH_{f} 51.0 kJ/mol), that the crystallisate consisting of Form I is contaminated with approximately 2 to 3% of Form II, which could also be seen thermomicroscopically.

### FT-IR, FT Raman spectroscope and X-ray diffractometry

FT-IR spectroscopy was carried out with a Bruker IFS 25 FT-IR spectrometer. For the production of the KBr compacts approximately 1 mg of sample was powdered with 270 mg of KBr. The spectra were recorded in transmission mode ranging from 4000 to 600 cm⁻¹. Resolution: 2 cm⁻¹ (50 interferograms).

FT Raman spectroscopy was carried out with Bruker RFS 100 FT Raman spectrometer, equipped with a diode-pumped Nd:YAG laser ( 1064 nm) and a liquid nitrogen cooled highly sensitive detector. The powdered samples were pressed into small aluminium fittings, the spectra were recorded at an initial capacity of 200 mW, resolution: 4 cm⁻¹ (50 interferograms).

X-ray powder diffractometry was carried out with a Simens X-ray diffractometer D-5000. Diffrac/AT with θ/θ goniometer, Cu_{Kα}-rays, nickel filter for monochromatisation, rotation of sample during measurement, scintillation counter, angular range 2° to 40° (2θ), steps of 0.01° (2θ), measuring time 2 secs.

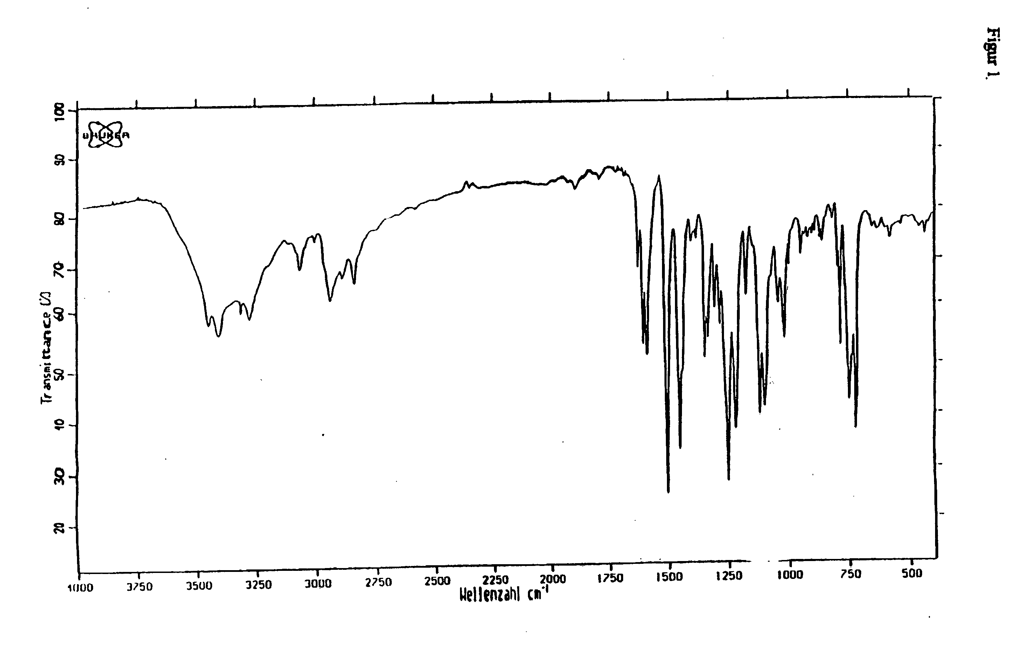

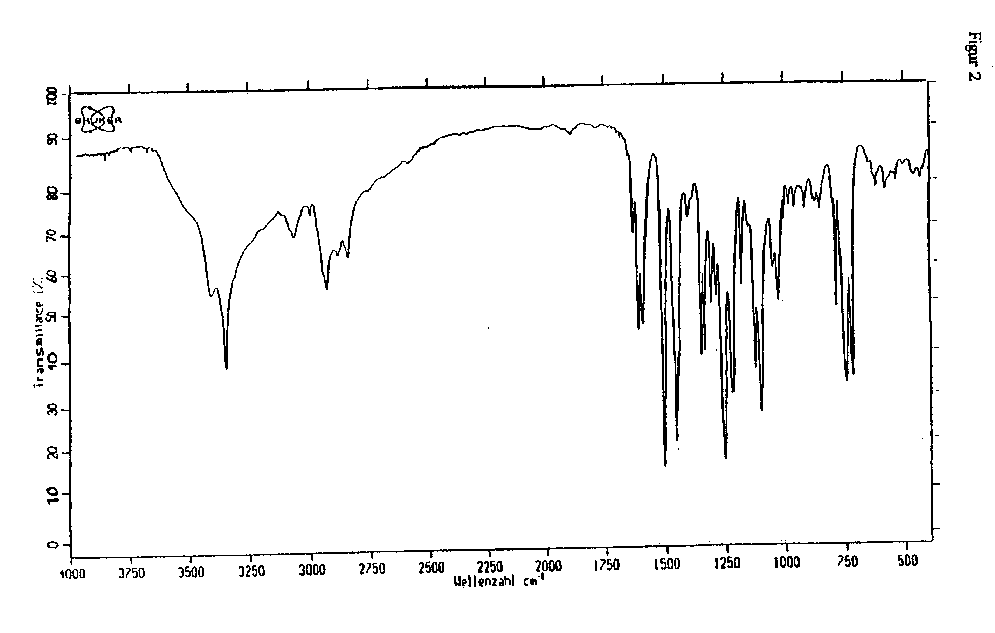

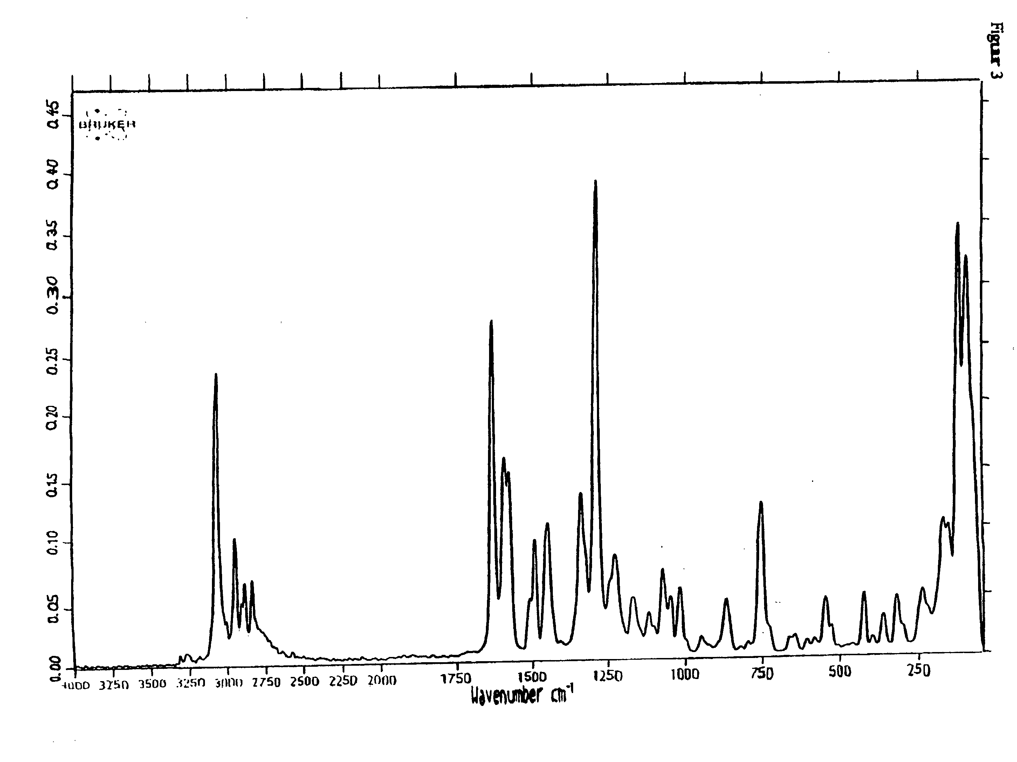

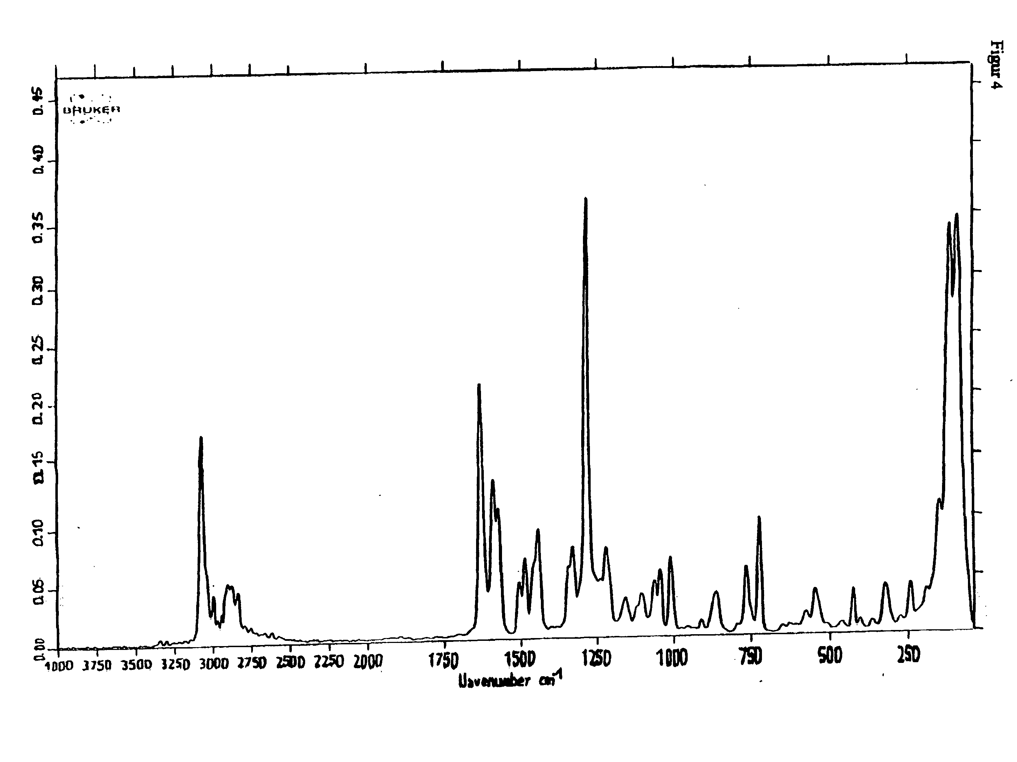

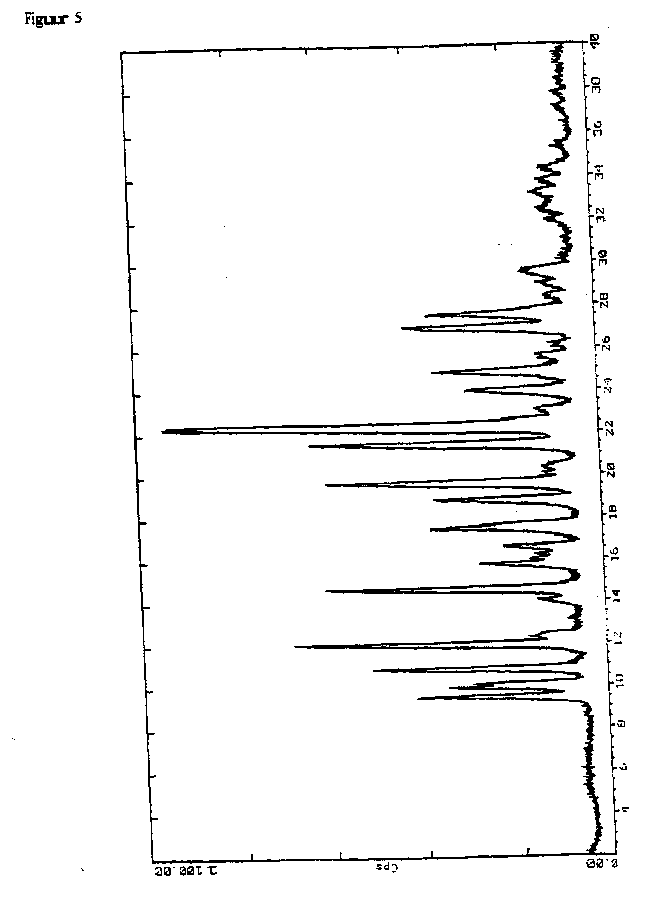

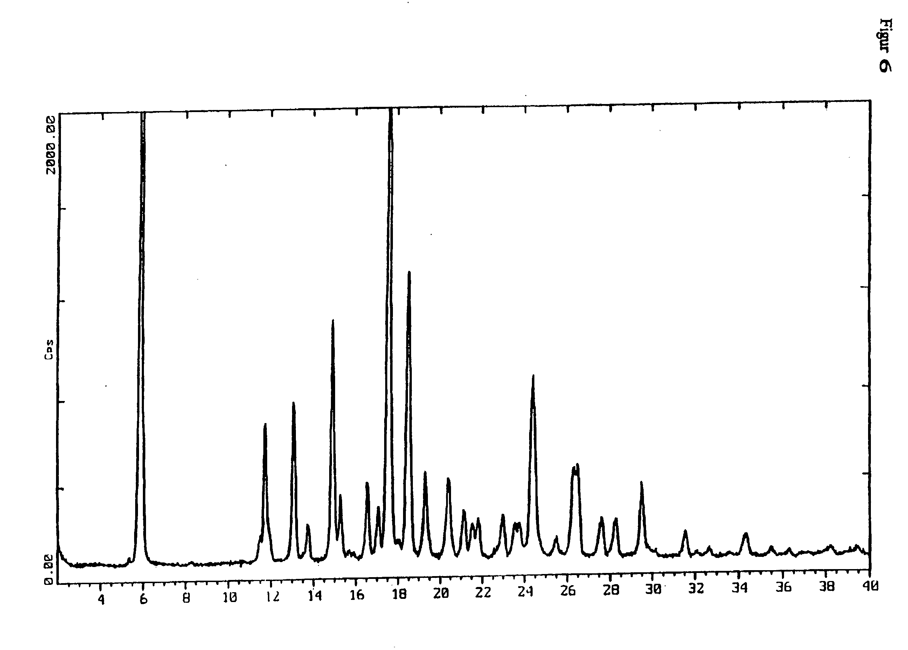

The IR spectra of both modifications show great differences in the stretching vibration range (Form I 3451 cm⁻¹; Form II 3345 cm⁻¹)(Fig. 1,2), which are caused by different hydrogen bridges. This corresponds to the Raman spectra differing only little. The biggest difference in the Raman spectra is at approximately 2942 and approximately 755 cm⁻¹ (Fig.3,4). The X-ray powder diffraction pattern of Form I has characteristic peaks occuring at 2θ = 9.5, 10.8, 12.0, 14.6, 19.6, 21.5, and 22.3 (Fig. 5) whereas the characteristic peaks of Form II occur at 2θ = 5.9, 14.9, 17.6, 18.5, and 24.4 (Fig. 6).

### Process for preparing Form I Carvedilol

### Example 1

Crude Carvedilol is prepared according to the procedure described in EP-B-0 004 920, in methanol. Crude Carvedilol (based on 300 g dry Carvedilol), 15 g CXA-coal and 2800 ml methanol are heated for 15 minutes under reflux in a three-neck-flask. The hot solution is filtered and washed with 300 ml hot methanol and heated under reflux again. Subsequently the solution is cooled down during half an hour to 30°C, stirred between 3 to 22 hours and cooled down slowly to 0°C in 3 ½ hours. After stirring the solution for additional two hours at 0°C the product is isolated, washed three times with 40 ml methanol and dried under vacuo at 60°C for 24 hours. 203-255 g of pure Form I are obtained and characterized as described before.
Form II can be obtained by an additional recrystallization process in isopropanol.

### Example 2

A 1:1 mixture of Form I and Form II was suspended in isopropanol and agitated with a magnetic stirrer for 18 h in a tightly sealed glass cylindar. During this time the temperature was repeatedly increased and lowered between 10 and 25°C. Subsequently the sample was filtered with a micro glass filter funnel (G3), dried and evacuated. The IR spectrum of this sample corresponds to Form 1. The DSC curve does not show a peak between 114-115°C, thus this is pure Form 1.

It will be understood that the specification and examples are illustrative but not limitative of the present invention and that other embodiments within the spirit and scope of the invention will suggest themselves to those skilled in the art.

## Claims

1. Modification of (±)1-(4-carbazolyloxy)-3-[2-(2-methoxyphenoxy)ethylamino]-2-propanole having the following X-ray diffraction pattern obtained with a Cu_{Kα} radiation at 2θ= 9.5, 10.8, 12.0. 14.5, 19.6, 21.5, 22.3, and an infrared spectrum having sharp peaks at 3451 cm⁻¹, wherein the melting point is about 123-126 °C.

2. Pharmacologically acceptable salts or optical active forms of the compound according to claim 1.

3. A process for preparing and isolating substantially pure form of the compound as claimed in claim 1.

4. The process of claim 3, wherein the rearrangement is carried out at a temperature between 25 and 35 °C for a time from 3 to 22 hours in methanol.

5. The process of claim 3 or 4, wherein the higher melting carvedilol modification is recovered at 0°C from the rearrangement reaction mixture.

6. A pharmaceutical composition comprising a substantially pure form of the stable modification of 1-(4-carbazolyloxy)-3-[2-(2-methoxyphenoxy)ethylamino]-2-propanole as the active ingredient as claimed in claim 1 together with one or more pharmaceutical acceptable carriers or adjuvants.

7. The use of a pharmaceutical composition as claimed in claim 5 for the manufacturing of a medicament for the prophylaxis or treatment of cardiac diseases.

## Patentansprüche

1. Modifikation von (±)1-(4-Carbazolyloxy)-3-[2-(2-methoxyphenoxy)ethylamino]-2-propanol, welche das folgende Röntgenbeugungsmuster, erhalten mit Cu_{Kα}-Strahlung, mit 2θ = 9,5, 10,8, 12,0, 14,5, 19,6, 21,5, 22,3, und ein Infrarotspektrum mit scharfen Peaks bei 3451 cm⁻¹ aufweist, wobei der Schmelzpunkt etwa 123-126°C beträgt.

2. Pharmakologisch verträgliche Salze oder optisch aktive Formen der Verbindung gemäß Anspruch 1.

3. Verfahren zur Herstellung und Isolierung der Verbindung gemäß Anspruch 1 in im wesentlichen reiner Form.

4. Verfahren gemäß Anspruch 3, wobei die Umwandlung bei einer Temperatur zwischen 25 und 35°C für 3 bis 22 Stunden in Methanol durchgeführt wird.

5. Verfahren gemäß Anspruch 3 oder 4, wobei die Carvediol-Modifikation mit dem höheren Schmelzpunkt bei 0°C aus dem Umwandlungsreaktionsgemisch gewonnen wird.

6. Arzneimittel umfassend eine im wesentlichen reine Form der stabilen Modifikation von 1-(4-Carbazolyloxy)-3-[2-(2-methoxyphenoxy)ethylamino]-2-propanol gemäß Anspruch 1 als Wirkstoff zusammen mit einem oder mehreren pharmazeutisch verträglichen Trägern und Hilfsstoffen.

7. Verwendung eines Arzneimittels gemäß Anspruch 5 zur Herstellung eines Medikaments zur Prophylaxe oder Behandlung von Herzkrankheiten.

## Revendications

1. Forme modifiée du (±) 1- (4- carbazolyloxy)-3-[ 2-(2- méthoxyphénoxy)éthylamino]-2-propanol possédant le diagramme de diffraction des rayons X suivant obtenu avec une raie Cu_{Kα} à 2θ = 9,5, 10,8, 12,0, 14,5, 19,6, 21,5, 22,3 et un spectre infrarouge présentant des pics nets à 3451 cm⁻¹ , dans laquelle le point de fusion est d'environ 123 - 126 °C.

2. Sels ou formes optiquement actives pharmacologiquement acceptables du composé selon la revendication 1.

3. Procédé pour préparer et isoler une forme pratiquement pure du composé selon la revendication 1.

4. Procédé selon la revendication 3, dans lequel le réarrangement s'effectue à une température comprise entre 25 et 35 °C sur une période de 3 à 22 heures dans le méthanol.

5. Procédé selon la revendication 3 ou 4, dans lequel la forme modifiée du Carvedilol à point de fusion plus élevé s'obtient par récupération à 0°C à partir du mélange de la réaction de réarrangement.

6. Composition pharmaceutique qui comprend une forme pratiquement pure de la forme modifiée stable du 1- (4- carbazolyloxy)-3-[ 2-(2- méthoxyphénoxy)éthylamino]-2-propanol à titre de principe actif selon la revendication 1, conjointement avec un ou plusieurs véhicules ou adjuvants pharmaceutiquement acceptables.

7. Utilisation d'une composition pharmaceutique selon la revendication 5 pour la fabrication d'un médicament pour la prophylaxie ou le traitement des maladies cardiaques.
